# FASCICULE DE BREVET EUROPEEN

(11) **EP 1 796 505 B1**
(45) Date de publication et mention de la délivrance du brevet: **09.09.2009**
(21) Numéro de dépôt: 05789907.2
(22) Date de dépôt: 08.07.2005
(51) Int. Cl.: A45D 40/26, A45D 34/04, A45D 40/00

(54) **KIT ET PROCEDE DE MAQUILLAGE**
MAKE-UP-KIT UND -VERFAHREN
MAKE-UP KIT AND METHOD

(30) Priorité: 05.10.2004 FR 0410509; 20.10.2004 US 619927 P
(43) Date de publication de la demande: 20.06.2007
(73) Titulaire: L'Oréal, 75008 Paris (FR)
(72) Inventeur: THEVENET, Ludovic, F-92340 BOURG LA REINE (FR)
(74) Mandataire: Tanty, François
(86) Numéro de dépôt international: PCT/FR2005/050558
(87) Numéro de publication internationale: WO 2006/037901

(56) Documents cités:
- EP-A- 1 043 018
- WO-A-03/020225
- FR-A- 2 825 247
- FR-A- 2 851 463
- US-A1- 2001 033 766
- US-A1- 2003 072 602

## Description

La présente invention concerne un procédé de maquillage tel que défini à la revendication 1.

Par "composition cosmétique", on désigne au sens de la présente invention une composition telle que définie dans la Directive 93/35/CEE du 14 juin 1993 modifiant la Directive 76/768/CEE. Les fonds de teint, rouges à lèvres et vernis à ongles sont des exemples de compositions cosmétiques.

Il existe un besoin pour bénéficier de nouveaux effets esthétiques dans le domaine du maquillage et l'invention vise à répondre à ce besoin.

### Kit de maquillage

Ci-après, on décrit un kit pour le maquillage d'un support (S) tel que la peau, les phanères ou les muqueuses, comportant :
- une première composition cosmétique (C₁) comportant des particules de fer métal, notamment de fer doux,
- une deuxième composition cosmétique (C₂), destinée à recouvrir ou à être recouverte par la première composition (C₁).

On décrit encore un kit pour le maquillage d'un support tel que la peau, les phanères ou les muqueuses, ou encore de faux ongles, comportant :
- une première composition cosmétique comportant des particules magnétiques mobiles sous l'effet d'un champ magnétique,
- une deuxième composition cosmétique destinée à recouvrir ou à être recouverte par la première composition, ce kit peut comporter également :

- un dispositif magnétique pour générer le champ magnétique permettant de déplacer et/ou de modifier l'orientation de tout ou partie des particules magnétiques lorsque la première composition est appliquée sous forme d'au moins une couche sur le support.

Par "mobile", il faut comprendre que l'orientation et/ou la position des particules peut être modifiée.

L'aspect de la première composition déposée sur le support dépend de l'orientation et/ou de l'emplacement des particules magnétiques. L'invention rend possible de créer de nouveaux effets de maquillage en permettant par exemple de réaliser des motifs en relief ou conférant une impression de relief ou divers autres motifs, géométriques ou non.

La deuxième composition peut être transparente. Lorsque la deuxième composition cosmétique est appliquée sur la première, elle peut permettre d'obtenir un effet de profondeur, de brillance, de lissage ou autre.

La deuxième composition peut comporter un agent de coloration, par exemple des pigments. Lorsque la deuxième composition est colorée, cela peut permettre par exemple de créer un fond coloré, la deuxième composition étant alors, par exemple, recouverte par la première.

Le kit peut notamment être utilisé pour le maquillage des lèvres ou des ongles.

Le kit peut comporter un dispositif magnétique pour générer un champ magnétique permettant de modifier l'aspect de la première composition, au moins immédiatement après son application sur le support.

### Première composition cosmétique

La première composition peut prendre un état empêchant tout nouveau changement d'orientation des particules magnétiques sous l'effet d'un champ magnétique après une durée de séchage donnée. C'est par exemple le cas d'un vernis à ongles. L'orientation des particules magnétiques peut encore dans certains cas être modifiée à tout moment, notamment lorsque la première composition ne sèche pas ou présente une durée de séchage très longue. Cela peut être le cas par exemple d'un fond de teint.

La première composition comporte des particules magnétiques, comme indiqué plus haut, lesquelles peuvent se présenter sous diverses formes.

### PARTICULES MAGNETIQUES

Par "particules magnétiques", encore appelées "corps magnétiques", on désigne des particules présentant une susceptibilité magnétique, c'est-à-dire sensible à l'action d'un champ magnétique et tendant par exemple à s'aligner sur les lignes de champ.

La première composition peut comporter à la fois des particules magnétiques et des particules non magnétiques.

La présence de particules magnétiques et de particules non magnétiques dans la composition peut permettre par exemple de créer de nouveaux effets optiques, modulables sous l'effet d'un champ magnétique.

De préférence, les particules magnétiques utilisées ne présentent pas d'aimantation rémanente en l'absence de champ magnétique.

Les particules magnétiques peuvent comporter tout matériau magnétique présentant une sensibilité aux lignes d'un champ magnétique, qu'il soit produit par un aimant permanent ou issu d'une induction, ce matériau étant par exemple choisi parmi le nickel, le cobalt, le fer, leurs alliages et oxydes, notamment Fe₃O₄, et aussi le gadolinium, le terbium, le dysprosium, l'erbium, leurs alliages et oxydes. Le matériau magnétique peut être de type "doux" ou "dur".

Les particules magnétiques peuvent présenter ou non une structure multicouche, comportant au moins une couche d'un matériau magnétique, tel que par exemple le fer, le nickel, le cobalt, leurs alliages et oxydes, notamment Fe₃O₄.

Les particules magnétiques sont de préférence asphériques, présentant par exemple une forme allongée. Ainsi, lorsque ces particules sont soumises au champ magnétique, elles tendent à s'orienter avec leur axe longitudinal dans l'alignement des lignes de champ, et subissent un changement d'orientation qui se traduit par un changement d'aspect de la première composition.

Lorsque les particules magnétiques sont sensiblement sphériques, de préférence leur aspect est inhomogène, de manière à ce qu'un changement d'orientation induise un changement d'aspect.

La quantité de particules magnétiques est suffisante pour que l'aspect de la composition puisse dépendre de leur orientation et/ou de leur emplacement.

La concentration en particules magnétiques est par exemple comprise entre environ 0,05 et environ 97 % en masse, mieux entre environ 0,1 et environ 95 % en masse, mieux entre 0,1 et 90 % en masse, par exemple de l'ordre de 3 % en masse. La dimension des particules magnétiques est par exemple comprise entre 1 nm et 700 µm, par exemple entre 1 µm et 500 µm, mieux encore entre environ 10 µm et environ 150 µm. Par "dimension", on désigne la dimension donnée par la distribution granulométrique statistique à la moitié de la population, dite D50.

Les particules magnétiques de la première composition peuvent comporter des pigments magnétiques. Des pigments convenant tout particulièrement sont les nacres comportant de l'oxyde de fer Fe₃O₄. Des pigments présentant des propriétés magnétiques sont par exemple ceux commercialisés sous les dénominations commerciales COLORONA BLACKSTAR BLUE, COLORONA BLACKSTAR GREEN, COLORONA BLACKSTAR GOLD, COLORONA BLACKSTAR RED, CLOISONNE NU ANTIQUE SUPER GREEN, MICRONA MATTE BLACK (17437), MICA BLACK (17260), COLORONA PATINA SILVER (17289) et COLORONA PATINA GOLD (117288) de la société MERCK ou bien encore FLAMENCO TWILIGHT RED, FLAMENCO TWILIGHT GREEN, FLAMENCO TWILIGHT GOLD, FLAMENCO TWILIGHT BLUE, TIMICA NU ANTIQUE SILVER 110 AB, TIMICA NU ANTIQUE GOLD 212 GB, TIMICA NU-ANTIQUE COPPER 340 AB, TIMICA NU ANTIQUE BRONZE 240 AB, CLOISONNE NU ANTIQUE GREEN 828 CB, CLOISONNE NU ANTIQUE BLUE 626 CB, GEMTONE MOONSTONE G 004, CLOISONNE NU ANTIQUE RED 424 CB, CHROMA-LITE BLACK (4498), CLOISONNE NU ANTIQUE ROUGE FLAMBE (code 440 XB), CLOISONNE NU ANTIQUE BRONZE (240 XB), CLOISONNE NU ANTIQUE GOLD (222 CB) et CLOISONNE NU ANTIQUE COPPER (340 XB) de la société ENGELHARD.

Les particules magnétiques peuvent être des fibres magnétiques.

### Fibres magnétiques

Le terme "fibres" désigne des corps généralement allongés, présentant par exemple un facteur de forme allant de 3,5 à 2 500 ou de 5 à 500, par exemple de 5 à 150. Le facteur de forme est défini par le rapport L/D, où L est la longueur de la fibre et D le diamètre du cercle dans lequel s'inscrit la plus grande section transversale de la fibre.

La section transversale des fibres peut s'inscrire par exemple dans un cercle de diamètre allant de 2 nm à 500 µm, par exemple allant de 100 nm à 100 µm, voire de 1 µm à 50 µm.

Les fibres peuvent présenter par exemple une longueur allant de 1 µm à 10 mm, par exemple de 0,1 mm à 5 mm, voire de 0,3 mm à 3,5 mm.

Les fibres peuvent présenter une masse allant par exemple de 0,15 à 30 deniers (masse en gramme pour 9 km de fil), par exemple de 0,18 à 18 deniers.

La forme en section transversale des fibres peut être quelconque, par exemple circulaire ou polygonale, notamment carrée, hexagonale ou octogonale.

La composition peut comporter des fibres pleines ou creuses, indépendantes ou liées entre elles, par exemple tressées.

La composition peut comporter des fibres ayant des extrémités épointées et/ou arrondies, par exemple par polissage.

Les fibres peuvent ne pas voir leur forme sensiblement modifiée lorsqu'elles sont introduites dans la composition, étant par exemple initialement rectilignes et suffisamment rigides pour conserver leur forme. En variante, les fibres peuvent présenter une souplesse leur permettant de se déformer sensiblement au sein de la composition.

Les fibres peuvent comporter une teneur non nulle, pouvant aller jusqu'à 100 %, d'un matériau magnétique choisi parmi les matériaux magnétiques doux, les matériaux magnétiques durs, notamment à base de fer, de zinc, de nickel, de cobalt ou de manganèse et leurs alliages et oxydes, notamment Fe₃O₄, les terres rares, le sulfate de baryum, les alliages de fer silicium, éventuellement chargés en molybdène, Cu₂MnAl, MnBi, ou un mélange de ceux-ci, cette liste n'étant pas limitative.

Lorsque la composition comporte des fibres contenant des particules magnétiques, ces dernières peuvent être présentes par exemple au moins à la surface de la fibre, voire à la surface des fibres uniquement, à l'intérieur de la fibre uniquement ou encore être dispersées au sein de la fibre de manière sensiblement homogène.

Les fibres peuvent comporter par exemple un coeur non magnétique avec une pluralité de particules magnétiques à sa surface.

Les fibres peuvent encore comporter une matrice synthétique contenant une pluralité de grains magnétiques dispersés en son sein.

Le cas échéant, une matière synthétique chargée de particules magnétiques peut elle-même être enrobée par une écorce non magnétique. Une telle écorce constitue par exemple une barrière isolant le ou les matériaux magnétiques du milieu ambiant et/ou peut amener de la couleur. Les fibres peuvent comporter un coeur magnétique monolithique et être enrobées par une écorce non magnétique, ou cela peut être l'inverse.

La composition peut comporter des fibres réalisées par extrusion ou co-extrusion d'une ou plusieurs matières polymériques, notamment thermoplastiques et/ou élastomères. L'une des matières extrudées peut contenir une charge de particules magnétiques dispersées.

La fibre peut comporter une matière synthétique choisie parmi les polyamides, PET, acétates, polyoléfines, notamment PE ou PP, PVC, polyester bloc amide, Rilsan^{®} plastifié, élastomères, notamment élastomères de polyester, élastomères de PE, élastomères de silicone, élastomères de nitrile ou un mélange de ces matériaux, cette liste n'étant pas limitative.

La composition peut contenir des fibres composites comportant un coeur magnétique enrobé au moins partiellement par au moins un matériau amagnétique, synthétique ou naturel. L'enrobage du coeur magnétique peut se faire par exemple par co-extrusion, autour du coeur, d'une écorce en un matériau non magnétique.

L'enrobage du coeur peut encore s'effectuer autrement, par exemple par polymérisation *in situ*.

Le coeur peut être monolithique ou comporter une charge de grains magnétiques dispersés dans une matrice.

La composition peut encore contenir des fibres composites obtenues par enrobage par une matière synthétique, chargée de particules magnétiques, d'un coeur amagnétique, synthétique ou naturel, le coeur étant composé par exemple d'une fibre de bois, de rayonne, de polyamide, d'une matière végétale, de polyoléfine, notamment de polyéthylène, de Nylon^{®}, de polyimide-amide, d'aramide, cette liste n'étant pas limitative.

La composition peut encore comporter des particules composites magnétiques, notamment un latex magnétique.

### Particules composites magnétiques

Une particule composite magnétique est un matériau composite constitué d'une matrice organique ou minérale et de grains magnétiques. Les particules composites magnétiques peuvent ainsi comporter à leur surface et/ou en leur sein des grains d'un matériau magnétique. Les particules composites peuvent être constituées d'un coeur magnétique enrobé d'une matrice organique ou minérale, ou inversement.

Les particules composites magnétiques comportent par exemple l'un des matériaux magnétiques précités.

La dimension des particules composites magnétiques est par exemple comprise entre 1 nm et 1 mm, mieux entre 100 nm et 500 µm, mieux encore entre 500 nm et 100 µm. Par "dimension", on désigne la dimension donnée par la distribution granulométrique statistique à la moitié de la population, dite D50.

La thèse de C. GOUBAULT, 23 Mars 2004, incorporée ici par référence, rappelle au chapitre 1 l'état de l'art en matière de particules composites magnétiques, et dresse une liste de procédés de préparation pouvant être utilisés pour préparer des particules composites magnétiques, à savoir une synthèse séparée des grains magnétiques et de la matrice, une synthèse des grains magnétiques au contact de la matrice ou une synthèse de la matrice en présence des grains magnétiques.

La société KISKER commercialise des particules magnétiques composites à matrice minérale, composée de silice. Les sociétés DYNAL, SERADYN, ESTAPOR et ADEMTECH proposent des particules magnétiques composites à matrice organique, susceptibles également d'être utilisées dans l'invention.

Plus particulièrement, la société ESTAPOR commercialise sous la référence M1-070/60 des latex magnétiques constitués de grains de ferrite uniformément répartis dans une matrice polystyrène, ce latex comportant 65 % d'oxyde de fer, le diamètre moyen des particules de polystyrène étant de 890 nm et la teneur massique en matières sèches de 10%.

### Ferrofluide

La composition peut comporter un ferrofluide, c'est-à-dire une suspension colloïdale stable de particules magnétiques, notamment de nanoparticules magnétiques.

Les particules, d'une taille par exemple de l'ordre de quelques dizaines de nanomètres, sont dispersées dans un solvant (eau, huile, solvant organique), soit à l'aide d'un tensioactif ou d'un agent dispersant, soit par des interactions électrostatiques.

Les ferrofluides sont par exemple préparés par broyage de ferrites ou autres particules magnétiques jusqu'à l'obtention de nanoparticules qui sont ensuite dispersées dans un fluide contenant un surfactant, lequel s'adsorbe sur les particules et les stabilise, ou par précipitation en milieu basique d'une solution d'ions métalliques.

Chaque particule du ferrofluide présente un moment magnétique déterminé par la taille de la particule et par la nature du matériau magnétique.

Sous l'action d'un champ magnétique, les moments magnétiques des particules tendent à s'aligner suivant les lignes de champ, avec apparition d'une aimantation non nulle dans le liquide. Si le champ est annulé, il n'y a pas d'hystérésis et l'aimantation s'annule.

Au-delà d'une valeur seuil de champ, on peut également provoquer des changements macroscopiques dans le liquide, par exemple l'apparition de pics ou une modification des propriétés rhéologiques.

La dénomination "ferrofluide" englobe également une émulsion de gouttelettes de ferrofluide dans un solvant. Chaque goutte contient alors des particules magnétiques colloïdales en suspension stable. Cela permet de disposer d'un ferrofluide dans tout type de solvant. La dimension des particules magnétiques en suspension dans le ferrofluide est par exemple comprise entre 1 nm et 10 µm, mieux entre 1 nm et 1 µm, mieux encore entre 1 nm et 100 nm. Par "dimension", on désigne la dimension donnée par la distribution granulométrique statistique à la moitié de la population, dite D50.

On peut citer notamment les ferrofluides commercialisés par la société LIQUIDS RESEARCH LTD sous les références :
- WHKS1S9 (A, B ou C), qui est un ferrofluide à base aqueuse comportant de la magnetite (Fe₃O₄), ayant des particules de 10 nm de diamètre.
- WHJS1 (A, B ou C), qui est un ferrofluide à base d'iso-paraffine et de particules de magnetite (Fe₃O₄) de 10 nm de diamètre.
- BKS25_dextran, qui est un ferrofluide à base aqueuse stabilisé par du dextran, comportant des particules de magnetite (Fe₃O₄) de 9 nm de diamètre.

### Chaînes de particules et/ou de fibres magnétiques

La composition peut comporter des agglomérats de particules ou fibres dont la plus grande dimension, par exemple la longueur, est par exemple comprise entre 1 nm et 10 mm, par exemple entre 10 nm et 5 mm, ou entre 100 nm et 1 mm, ou encore entre 0,5 µm et 3,5 mm, par exemple entre 1 µm et 150 µm. La dimension désigne celle donnée par la distribution granulométrique statistique à la moitié de la population, dite D50.

Des chaînes de particules magnétiques peuvent être obtenues par exemple en assemblant des particules magnétiques colloïdales, comme cela est décrit dans les publications "Permanently linked monodisperse paramagnetic chains", E.M. Furst, C. Suzuki, M. Fermigier, A.P. Gast, Langmuir, 14, 7334-7336 (1998), "Suspensions de particules magnétiques", M. Fermigier, Y. Grasselli, Bulletin de la SFP (105) juillet 96, et "Flexible magnetic filaments as micromechanical sensors", C. Goubault, P. Jop, M. Fermigier, J. Baudry, E. Bertrand, J. Bibette, Phys. Rev. Lett., 91, 26, 260802-1 à 260802-4 (2003), dont les contenus sont incorporés par référence.

Il est notamment décrit dans ces articles comment procéder pour obtenir des chaînes de particules de latex magnétiques comportant une matrice de polystyrène contenant des grains d'oxyde de fer et fonctionnalisées en surface, liées entre elles de façon permanente suite à une réaction chimique, notamment des liaisons covalentes entre les surfaces des particules adjacentes ; il est également décrit un procédé d'obtention de chaînes de gouttelettes d'émulsion de ferrofluides, liées entre elles par interactions de nature physique. La longueur ainsi que le diamètre des chaînes permanentes ainsi obtenues peuvent être contrôlés. De telles chaînes magnétiques constituent des objets magnétiques anisotropes orientables et déplaçables sous l'effet d'un champ magnétique.

Les dimensions des chaînes magnétiques peuvent répondre aux mêmes conditions que les fibres magnétiques.

La première composition peut comporter par exemple au moins un agent de coloration goniochromatique qui permet d'observer un changement de couleur en fonction de l'angle d'observation. Cet agent de coloration goniochromatique peut être magnétique ou non.

Lorsque la première composition comporte des particules magnétiques d'une certaine couleur et un agent de coloration goniochromatique, ce dernier peut être choisi de manière à ce que le trajet de couleur passe sensiblement par la couleur des particules magnétiques.

Cela peut permettre par exemple de rendre plus difficilement détectables les particules magnétiques à défaut d'une orientation de celles-ci sous l'effet d'un champ magnétiques.

Cela peut permettre également de ne faire bien apparaître le motif induit par l'orientation des particules magnétiques que dans certaines conditions d'observation et/ou d'éclairage du support maquillé.

### AGENTS DE COLORATION GONIOCHROMATIQUES

Par "agent de coloration goniochromatique", on désigne au sens de la présente invention un agent de coloration permettant d'obtenir, lorsque la composition est étalée sur un support, un trajet de couleur dans le plan a*b* de l'espace colorimétrique CIE 1976 qui correspond à une variation Dh de l'angle de teinte h d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la première composition a été étalée à l'état fluide avec une épaisseur de 300 µm au moyen d'un étaleur automatique sur une carte de contraste de marque ERICHSEN et de référence Typ 24/5, la mesure étant effectuée sur le fond noir de la carte.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche étant réalisée par exemple à partir d'au moins un matériau choisi dans le groupe constitué par les matériaux suivants : MgF₂, CeF₃, ZnS, ZnSe, Si, SiO₂ Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

La structure multicouche peut présenter ou non, par rapport à une couche centrale, une symétrie au niveau de la nature chimique des couches empilées. Selon l'épaisseur et la nature des différentes couches, on obtient différents effets.

Des exemples de structures multicouche interférentielles symétriques sont par exemple les structures suivantes : Fe₂O₃/SiO₂/Fe₂O₃/SiO₂/Fe₂O₃, un pigment ayant cette structure étant commercialisé sous la dénomination SICOPEARL par la société BASF ; MoS₂/SiO₂/mica-oxyde/SiO₂/MoS₂; Fe₂O₃/SiO₂/mica-oxyde/SiO₂/Fe₂O₃; TiO₂/SiO₂/TiO₂ et TiO₂/Al₂O₃/TiO₂, des pigments ayant ces structures étant commercialisés sous la dénomination XIRONA par la société MERCK (Darmstadt).

Les agents de coloration à cristaux liquides comprennent par exemple des silicones ou des éthers de cellulose sur lesquels sont greffés des groupes mésomorphes. Comme particules goniochromatiques à cristaux liquides, on peut utiliser par exemple celles vendues par la société CHENIX ainsi que celles commercialisées sous la dénomination HELICONE^{®} HC par la société WACKER.

Comme agent de coloration goniochromatique, on peut encore utiliser certaines nacres, des pigments à effets sur substrat synthétique, notamment substrat type alumine, silice, borosilicate, oxyde de fer, aluminium, ou des paillettes holographiques interférentielles issues d'un film de polytéréphthalate.

Le matériau peut en outre comporter des fibres goniochromatiques dispersées. De telles fibres pourront présenter une longueur inférieure à 80 µm par exemple.

La première composition peut également comporter au moins un pigment diffractant, lequel peut présenter des propriétés magnétiques le cas échéant.

### PIGMENTS DIFFRACTANTS

Par "pigment diffractant", on désigne au sens de la présente invention un pigment capable de produire une variation de couleur selon l'angle d'observation lorsqu'éclairé par de la lumière blanche, en raison de la présence d'une structure qui diffracte la lumière.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel.

On pourra utilement se reporter concernant la structure des pigments diffractant à l'article "Pigments Exhibiting Diffractive Effects" d'Alberto Argoitia and Matt Witzman, 2002, Society of Vacuum coaters, 45th Annual Technical Conference Proceedings 2002.

Le pigment diffractant peut être réalisé avec des motifs ayant différents profils, notamment triangulaires, symétriques ou non, en créneaux, de largeur constante ou non, sinusoïdaux.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette.

Une même particule de pigment peut comporter deux réseaux de diffraction croisés, perpendiculaires ou non.

Le pigment diffractant peut présenter une structure multicouche comportant une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : MgF₂, SiO₂, Al₂O₃, AlF₃, CeF₃, LaF₃, NdF₃, SmF₂, BaF₂, CaF₂, LiF et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr et leurs matériaux, associations ou alliages. Un tel matériau réfléchissant peut, seul, constituer le pigment diffractant qui sera alors monocouche.

En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : MgF₂, SiO, SiO₂, Al₂O₃, TiO₂, WO, AIN, BN, B₄C, WC, TiC, TiN, N₄Si₃, ZnS, des particules de verre, des carbones de type diamant et leurs associations.

En variante, le pigment diffractant peut être composé d'un matériau diélectrique ou céramique préformé tel qu'un minéral en lamelle naturelle, par exemple du mica peroskovite ou du talc, ou des lamelles synthétiques formées à partir de verre, d'alumine, de SiO₂, de carbone, d'un oxyde de fer/mica, de mica recouvert de BN, de BC, de graphite, d'oxychlorure de bismuth, et leurs associations.

A la place d'une couche d'un matériau diélectrique, d'autres matériaux améliorant les propriétés mécaniques peuvent convenir. De tels matériaux peuvent comporter du silicone, des silicides métalliques, des matériaux semi-conducteurs formés à partir d'éléments des groupes III, IV et V, des métaux ayant une structure cristalline cubique centrée, des compositions ou matériaux de cermet, des verres semi-conducteurs, et leurs associations variées.

Le pigment diffractant utilisé peut notamment être choisi parmi ceux décrits dans la demande de brevet américain US 2003/0031870 publiée le 13 février 2003.

Un pigment diffractant peut comporter par exemple la structure suivante: MgF₂/Al/MgF₂, un pigment diffractant ayant cette structure étant commercialisé sous la dénomination SPECTRAFLAIR 1400 Pigment Silver par la société FLEX PRODUCTS, ou SPECTRAFLAIR 1400 Pigment Silver FG. La proportion en poids du MgF₂ peut être comprise entre 80 et 95 % du poids total du pigment.

La quantité de pigment diffractant peut varier, en poids par rapport au poids total de la première composition, par exemple de 0,1 à 5 %.

La dimension du pigment diffractant peut être comprise par exemple entre 5 et 200 µm, mieux entre 5 et 100 µm, par exemple entre 5 et 30 µm.

L'épaisseur des particules de pigment diffractant peut être inférieure ou égale à 3 µm, mieux 2 µm, par exemple de l'ordre de 1 µm.

### PARTICULES REFLECHISSANTES

La première composition peut comprendre par exemple des particules réfléchissantes, notamment des paillettes, entre autres, magnétiques ou non.

Par "particules réfléchissantes", on désigne au sens de la présente invention des particules dont la taille, la structure, notamment l'épaisseur de la ou des couches qui la constituent et leurs natures physique et chimique, et l'état de surface, leur permettent de réfléchir la lumière incidente. Cette réflexion peut, le cas échéant, posséder une intensité suffisante pour créer à la surface de la composition ou du mélange, lorsque celui-ci est appliqué sur le support à maquiller, des points de surbrillance visibles à l'oeil nu, c'est-à-dire des points plus lumineux qui contrastent avec leur environnement en semblant briller.

Les particules réfléchissantes peuvent être sélectionnées de manière à ne pas altérer significativement l'effet de coloration généré par les agents de coloration qui leur sont associés et plus particulièrement de manière à optimiser cet effet en terme de rendu de couleur. Elles peuvent plus particulièrement posséder une couleur ou un reflet jaune, rose, rouge, bronze, orangé, brun, or et/ou cuivré.

Les particules réfléchissantes peuvent être présentes dans la première composition à une teneur allant de 0,5 % à 60 % par rapport au poids total de la première composition, notamment de 1 % à 30 % en poids, en particulier de 3 % à 10 % en poids.

Ces particules peuvent présenter des formes variées, notamment être en forme de plaquettes ou globulaires, en particulier sphériques.

Les particules réfléchissantes, quelle que soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, notamment des oxydes de titane ou de fer obtenus par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou matériau métallique. Le substrat peut être monomatière, multimatériau, organique et/ou inorganique.

Plus particulièrement, il peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.

Le matériau réfléchissant peut comporter une couche de métal ou d'un matériau métallique.

Des particules réfléchissantes sont décrites notamment dans les documents JP-A-09188830, JP-A-10158450, JP-A-10158541, JP-A-07258460 et JP-A-05017710.

Toujours à titre d'exemple de particules réfléchissantes comportant un substrat minéral enrobé d'une couche de métal, on peut citer également les particules comportant un substrat de borosilicate enrobé d'argent, encore appelées "nacres blanches".

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes quelle que soit leur forme, peuvent également être choisies parmi les particules à substrat synthétique enrobé au moins partiellement d'au moins une couche d'au moins un matériau métallique, notamment un oxyde métallique, choisi par exemple parmi les oxydes de titane, notamment TiO₂, de fer notamment Fe₂O₃, d'étain, de chrome, le sulfate de baryum et les matériaux suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS^{®} par la société ENGELHARD.

La première composition selon l'invention peut comprendre au moins une nacre, magnétique ou non.

### NACRES

Par "nacre", il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être introduites dans la première composition, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica); les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

La première composition peut comporter par exemple au moins une charge, magnétique ou non.

### CHARGES

Par "charge", on désigne des particules de toute forme, insolubles dans le milieu de la composition, quelle que soit la température à laquelle la composition est fabriquée. Une charge peut servir notamment à modifier la rhéologie ou la texture de la composition. La nature et la quantité des particules pourra dépendre des propriétés mécaniques et des textures recherchées.

A titre d'exemple de charges, on peut citer, entre autres, le talc, le mica, la silice, le kaolin, la séricite, les poudres de polyamide, de polyoléfine, par exemple de polyéthylène, de polytétrafluoroéthylène, de polyméthacrylate de méthyle, de polyuréthane, les poudres d'amidon et les billes de résine de silicone.

Les charges peuvent être destinées à créer, entre autres, un effet de flou, notamment dans le cas d'un fond de teint, afin de dissimuler des imperfections de la peau.

La première composition peut encore comporter des colorants, pigments organiques ou laques.

### COLORANTS, PIGMENTS ORGANIQUES ET LAQUES

Les colorants peuvent être liposolubles ou hydrosolubles.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave et le bleu de méthylène.

Les colorants peuvent par exemple représenter de 0,1 à 20 % du poids de la première ou de la deuxième composition, voire de 0,1 à 6 %, lorsque présents.

Les laques ou pigments organiques peuvent être choisis parmi les matériaux ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les matériaux chimiques correspondant à chacune des matières colorantes organiques citées précédemment sont mentionnés dans l'ouvrage "International Cosmetic Ingredient Dictionnary and Handbook", Edition 1997, pages 371 à 386 et 524 à 528, publié par "The Cosmetic, Toiletry, and Fragrance Association", dont le contenu est incorporé dans la présente demande par référence.

La première composition peut comporter un pigment composite.

### PIGMENTS COMPOSITES

Le pigment composite peut être composé notamment de particules comportant :
- un noyau inorganique, magnétique ou non,
- au moins un enrobage au moins partiel d'au moins une matière colorante organique.

Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par "en forme de plaquettes", on désigne des particules dont le rapport de la plus grande dimension à l'épaisseur est supérieur ou égal à 5.

Un pigment composite peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

Le noyau inorganique du pigment composite peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

Le rapport de la plus grande dimension du noyau à sa plus petite dimension peut être compris entre 1 et 50.

Le noyau inorganique peut présenter une dimension comprise entre environ 1 nm et environ 100 nm, voire entre environ 5 nm et environ 75 nm, par exemple entre environ 10 nm et environ 50 nm.

Le noyau inorganique peut être réalisé dans un matériau choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges.

Les oxydes de titane, notamment TiO₂, de fer, notamment Fe₂O₃, de cérium, de zinc et d'aluminium, les silicates, notamment les aluminosilicates et les borosilicates conviennent tout particulièrement.

Le noyau inorganique peut présenter une surface spécifique, mesurée par la méthode BET, comprise par exemple entre environ 1 m²/g et environ 1000 m²/g, mieux entre environ 10 m²/g et environ 600 m²/g, par exemple entre environ 20 m²/g et environ 400 m²/g_{.}

Le noyau inorganique peut être coloré, le cas échéant.

La matière colorante organique peut être telle que définie plus haut.

Le liant du pigment composite peut être de tout type dès lors qu'il permet à la matière colorante organique d'adhérer à la surface du noyau inorganique.

Le liant peut notamment être choisi parmi une liste non limitative comprenant les matériaux siliconés, les matériaux polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

L'agent de coloration peut comporter une matière colorante photochrome ou agent photochrome.

### AGENTS PHOTOCHROMES

D'une manière générale, un agent de coloration photochrome est un agent colorant ayant la propriété de changer de teinte lorsqu'il est éclairé par la lumière ultraviolette et de rétablir sa couleur initiale lorsqu'il n'est plus éclairé par cette lumière ou encore de passer d'un état non coloré à un état coloré et inversement. En d'autres termes, un tel agent présente des teintes différentes selon qu'il est éclairé par de la lumière contenant une certaine quantité de radiations UV comme dans la lumière solaire ou de la lumière artificielle.

On pourra utilement se référer aux exemples d'agents photochromes décrits dans EP 1 410 786.

### AGENTS THERMOCHROMES

On peut par exemple utiliser l'agent thermochrome de la référence KROMAFAST YELLOW 5GX 02 vendu par la société KROMACHEM LTD.

### AUTRES AGENTS DE COLORATION

La première composition peut encore comporter des composés piézochromes, notamment tribochromes ou solvatochromes.

### AUTRES COMPOSANTS

Typiquement, la composition cosmétique comporte un milieu physiologiquement acceptable. Par "milieu physiologiquement acceptable", on désigne un milieu non toxique et susceptible d'être appliqué sur la peau, les phanères ou les lèvres d'êtres humains. Le milieu physiologiquement acceptable est généralement adapté à la nature du support sur lequel doit être appliquée la composition ainsi qu'à la forme sous laquelle la composition est conditionnée.

La première composition peut comporter d'autres ingrédients que ceux décrits plus haut, notamment au moins un solvant, une phase grasse, un polymère filmogène et/ou un actif dermatologique ou cosmétique, notamment en fonction de la forme galénique.

### SOLVANTS

La première composition peut comporter au moins un solvant aqueux ou organique, notamment un solvant organique volatil, par exemple une huile volatile.

Au sens de la présente invention, on entend par « solvant volatil», un solvant, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm Hg), et de préférence allant de 1,3 Pa à 13 000 Pa (0,01 à 100 mm Hg), et préférentiellement allant de 1,3 Pa à 1300 Pa (0,01 à 10 mm Hg).

Lorsque la première composition comprend un ou plusieurs solvants organiques, ces solvants peuvent être présents en une teneur allant de 0,1 % à 99 %, par rapport au poids total de la composition concernée.

D'une manière générale, la quantité de solvant(s), notamment organique(s), dépendra de la nature du support sur lequel la composition est destinée à être appliquée.

La première composition peut comporter au moins un solvant volatil constitué par une huile volatile.

L'huile peut être une huile siliconée ou une huile hydrocarbonée, ou comporter un mélange de telles huiles.

Au sens de la présente invention, on entend par « huile siliconée », une huile comprenant au moins un atome de silicium, et notamment au moins un groupe Si-O.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C8-C16 (appelées aussi isoparaffines) comme l'isododécane (encore appelé 2,2,4,4,6-pentaméthylheptane), l'isodécane, l'isohexadécane, et par exemple les huiles vendues sous les noms commerciaux d'ISOPARS^{®} ou de PERMETHYLS^{®}.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées tels que le nonafluorométhoxybutane ou le perfluorométhylcyclopentane, et leurs mélanges.

La composition peut comprendre entre 0,01 % et 95 % en poids d'huile volatile, par rapport au poids total de la composition, mieux entre 1 % et 75 % en poids.

La première composition peut comporter au moins un solvant organique choisi dans la liste suivante :
- les cétones liquides à température ambiante, tels que le méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les alcanes liquides à température ambiante tels que le décane, l'heptane, le dodécane, le cyclohexane.

La première composition peut aussi comprendre de l'eau ou un mélange d'eau et de solvants organiques hydrophiles couramment utilisés en cosmétique comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La première composition peut, en outre, contenir des éthers en C₂ et des aldéhydes en C₂-C₄ hydrophiles. L'eau ou le mélange d'eau et de solvants organiques hydrophiles peut être présent dans la première et/ou la deuxième composition en une teneur allant par exemple de 0 % à 90 %, notamment 0,1 % à 90 % en poids et de préférence de 0 % à 60 % en poids, notamment 0,1 % à 60 % en poids, par rapport au poids total de la composition.

### PHASE GRASSE

La première composition, par exemple lorsqu'elle est destinée à être appliquée sur les lèvres, peut comporter une phase grasse et notamment au moins un corps gras liquide à température ambiante (25 °C) et/ou un corps gras solide à température ambiante tel que les cires, les corps gras pâteux, les gommes et leurs mélanges. La phase grasse peut, en outre, contenir des solvants organiques lipophiles.

La première composition peut présenter par exemple une phase grasse continue, pouvant contenir moins de 5 % d'eau, notamment moins de 1 % d'eau par rapport à son poids total et en particulier être sous forme anhydre.

Comme corps gras liquides à température ambiante, appelés souvent "huiles", on peut citer : les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité, de lanoline, de lanoline acétylée ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; l'isonanoate d'isononyle, le lanolate d'isopropyle, le trimellilate de tridécyle, le malate de diisostéaryle ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges. Les huiles peuvent être présentes en une teneur allant de 0,01 à 90 %, et mieux de 0,1 à 85 % en poids, par rapport au poids total de la composition.

La présence d'une phase huileuse peut conférer de la brillance et présenter par exemple un indice de réfraction compris entre 1,47 et 1,51, mieux entre 1,48 et 1,50. L'indice de réfraction est mesuré à température ambiante (25 °C), à l'aide d'un réfractomètre.

La première composition peut comporter un corps gras pâteux, une cire ou une gomme.

Les corps gras pâteux sont généralement des composés hydrocarbonés avec un point de fusion compris entre 25 et 60 °C, de préférence entre 30 et 45 °C, et/ou une dureté comprise entre 0,001 et 0,5 MPa, de préférence entre 0,005 et 0,4 MPa, comme les lanolines et leurs dérivés.

Les cires peuvent être solides à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En particulier, les cires peuvent présenter une température de fusion supérieure à 25 °C et mieux supérieure à 45 °C. Les cires peuvent être hydrocarbonées, fluorées et/ou siliconées et être d'origine végétale, minérale, animale et/ou synthétique. Comme cires utilisables on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone. La composition peut contenir de 0 à 50 % en poids de cires, par rapport au poids total de la composition, voire de 1 à 30 % en poids.

Les gommes pouvant être utilisées sont généralement des polydiméthylsiloxanes (PDMS) à haut poids moléculaire ou des gommes de cellulose ou des polysaccharides.

### POLYMERES FILMOGENES

La première composition peut encore comporter, par exemple, un polymère filmogène, notamment dans le cas d'un mascara ou d'un vernis à ongles. "Polymère filmogène" désigne un polymère apte à former à lui seul ou en présence d'un agent auxiliaire de filmification, un film continu et adhérent sur un support, notamment sur les matières kératiniques.

Parmi les polymères filmogènes utilisables dans la première composition selon l'invention, on peut citer entre autres les polymères synthétiques, de type radicalaire ou de type polycondensat, les polymères d'origine naturelle, tels que la nitrocellulose ou les esters de cellulose, et leurs mélanges.

Les polymères filmogènes de type radicalaire peuvent être notamment des polymères ou des copolymères vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés α,β-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène.

Parmi les polycondensats filmogènes, on peut citer les polyuréthanes, les polyesters, les polyesters amides, les polyamides, et les polyurées, cette liste n'étant pas limitative.

Les polymères d'origine naturelle, éventuellement modifiés, peuvent être choisis parmi la résine shellac, la gomme de sandaraque, les dammars, les élémis, les copals, les polymères cellulosiques, tels que la nitrocellulose, l'éthylcellulose ou les esters de nitrocellulose choisis, par exemple, parmi l'acétate de cellulose, l'acétobutyrate de cellulose, l'acétopropionate de cellulose, et leurs mélanges.

Le polymère filmogène peut être présent sous la forme de particules solides en dispersion aqueuse ou huileuse, connue généralement sous le nom de latex ou pseudolatex. Le polymère filmogène peut comporter une ou plusieurs dispersions stables de particules de polymères généralement sphériques d'un ou plusieurs polymères, dans une phase grasse liquide physiologiquement acceptable. Ces dispersions sont généralement appelées NAD (Non-Aqueous Dispersion) de polymère par opposition à des latex qui sont des dispersions aqueuses de polymère. Ces dispersions peuvent notamment se présenter sous forme de nanoparticules de polymères en dispersion stable dans ladite phase grasse. Les nanoparticules sont de préférence d'une taille comprise entre 5 et 600 nm. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90^{®}, NEOCRYL A-1070^{®}, NEOCRYL A-1090^{®}, NEOCRYL BT-62^{®,} NEOCRYL A-1079^{®}, NEOCRYL A-523^{®} par la société AVECIA-NEORESINS, DOW LATEX 432^{®} par la société DOW CHEMICAL, DAITOSOL 5000 AD^{®} par la société DAITO KASEI KOGYO; ou encore les dispersions aqueuses de polyuréthane vendues sous les dénominations NEOREZ R-981^{®}, NEOREZ R-974^{®} par la société AVECIA-NEORESINS, les AVALURE UR-405^{®}, AVALURE UR-410^{®}, AVALURE UR-425^{®}, AVALURE UR-450^{®}, SANCURE 875^{®}, SANCURE 861^{®}, SANCURE 878^{®}, SANCURE 2060^{®} par la société GOODRICH, IMPRANIL 85^{®} par la société BAYER, AQUAMERE H-1511^{®} par la société HYDROMER ; les sulfopolyesters vendus sous le nom de marque Eastman AQ par la société Eastman Chemical Products.

La première composition selon l'invention peut comprendre également un agent auxiliaire de filmification favorisant la formation d'un film avec le polymère filmogène.

### ACTIFS

La première composition peut comporter au moins un actif cosmétique ou dermatologique. Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...), autobronzants. Ces actifs peuvent être utilisés par exemple à des concentrations de 0 à 20 % et notamment de 0,001 à 15 % par rapport au poids total de la composition.

La première composition peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, les colorants ou leurs mélanges.

La première composition selon l'invention peut comprendre, selon le type d'application envisagée, les constituants classiquement utilisés dans les domaines considérés, qui sont présents en une quantité appropriée à la forme galénique souhaitée.

### FORMES GALENIQUES

La première composition peut se présenter sous diverses formes, en fonction de sa destination. La première composition peut ainsi se présenter sous toute forme galénique normalement utilisée pour une application topique et notamment sous forme anhydre, sous forme d'une solution huileuse ou aqueuse, d'un gel huileux ou aqueux, d'une émulsion huile dans eau, eau dans huile, cire dans eau ou eau dans cire, d'une émulsion multiple, d'une dispersion d'huile dans de l'eau grâce à des vésicules situées à l'interface huile/eau.

La première composition peut se présenter sous la forme d'une poudre, ou encore d'un gel.

### Deuxième composition cosmétique

La deuxième composition peut être transparente, éventuellement incolore, et être destinée par exemple à l'application sur les lèvres, les ongles ou la peau. Elle peut comprendre l'un au moins des composants décrits plus haut pour la première composition.

La deuxième composition peut comporter au moins un agent de coloration, par exemple l'un de ceux énumérés ci-dessus.

La deuxième composition peut présenter, le cas échéant, des propriétés magnétiques, mais dans de nombreux exemples de mise en oeuvre de l'invention, seule la première composition présente des propriétés magnétiques.

La deuxième composition peut être destinée à recouvrir la première composition ou à être recouverte par la première composition.

Lorsque la deuxième composition est colorée, sa couleur peut contraster ou non avec celle de la première composition.

La deuxième composition peut se présenter sous une même forme galénique que la première, et la description qui précède, concernant le milieu de la composition et les actifs éventuels, s'applique également à la deuxième composition.

### Dispositifs magnétiques

Le dispositif magnétique peut comporter un aimant permanent ou un électroaimant, alimenté par exemple par au moins une pile ou un accumulateur. Dans ce dernier cas, le dispositif magnétique peut comporter un interrupteur permettant d'alimenter sélectivement l'électroaimant en électricité.

Le dispositif magnétique peut être agencé pour créer un champ magnétique dont l'orientation varie dans le temps. Lorsque le dispositif magnétique comporte un aimant, le dispositif peut par exemple comporter un moteur permettant d'entraîner en rotation l'aimant. En variante, le dispositif magnétique peut comporter plusieurs solénoïdes disposés de manière à générer, lorsqu'alimentés séquentiellement en électricité, un champ magnétique tournant.

Un champ magnétique rotatif peut permettre par exemple d'obtenir un motif présentant une symétrie de révolution, par exemple un motif donnant l'impression d'une sphère en relief.

Le ou les électroaimants peuvent être alimentés en permanence ou par intermittence, au choix de l'utilisateur. En particulier, le dispositif magnétique peut être agencé de telle sorte que le ou les électroaimants puissent ne pas être alimentés tant que le dispositif magnétique n'est pas positionné correctement près du support revêtu de la première composition.

Le champ magnétique est par exemple d'au moins 50 mT, voire d'au moins 66 mT.

De manière à rendre plus facile l'application du champ magnétique, le dispositif magnétique peut comporter un organe permettant de le positionner relativement au support sur lequel la première composition a été déposée. Cela peut permettre par exemple d'éviter que le dispositif magnétique ne vienne accidentellement en contact avec la composition et/ou de centrer le motif réalisé sur la région concernée.

Dans un exemple de mise en oeuvre de l'invention, le dispositif magnétique est solidaire d'un applicateur, par exemple celui servant à l'application de la première composition cosmétique. Cela peut permettre de réduire le nombre d'objets manipulés par l'utilisateur et faciliter le maquillage.

Dans un autre exemple de mise en oeuvre de l'invention, le dispositif magnétique comporte un aimant monté à une première extrémité d'une tige dont la deuxième extrémité est reliée à un organe de préhension d'un applicateur, par exemple celui servant à l'application de la première composition cosmétique.

Le champ magnétique peut encore être exercé au moyen d'une structure magnétique, notamment souple, comportant une alternance de pôles N et S. Une telle structure peut permettre par exemple de réaliser des motifs répétitifs sur la première composition, par exemple des rayures.

### Procédé de maquillage

L'invention concerne un procédé de maquillage tel que défini à la revendication 1.

L'exposition au champ magnétique peut avoir lieu avant et/ou après l'application de la deuxième composition sur le support ou sur la première composition.

Le champ magnétique peut être appliqué de manière à former au moins un motif sur la première composition, celui-ci étant par exemple lié à la géométrie des lignes de champ.

Comme indiqué ci-dessus, la deuxième composition cosmétique appliquée sur la première peut permettre par exemple d'obtenir un effet de profondeur, de brillance, de lissage ou autre. Cette deuxième composition peut être transparente. La deuxième composition peut encore être recouverte par la première afin par exemple de créer un fond coloré.

La deuxième composition est par exemple destinée à l'application sur les lèvres ou les ongles.

Le champ magnétique peut être appliqué de manière à modeler la clarté et/ou la couleur d'une région au moins du visage ou du corps sur laquelle la première composition a été appliquée.

Par exemple, quand la composition cosmétique est un fond de teint, l'orientation des particules sous l'effet du champ magnétique rend possible de modifier la clarté de la composition et de modeler ainsi l'aspect du visage selon les régions exposées au champ magnétique, afin notamment de réaliser un maquillage du type clair/obscur. Le champ magnétique peut par exemple être appliqué de manière à assombrir les côtés du visage afin de le faire paraître plus mince qu'il n'est réellement.

Le champ magnétique peut être appliqué jusqu'à obtenir un aspect figé de la première composition, c'est-à-dire que l'aspect de celle-ci cesse d'évoluer même si le champ magnétique perdure. En variante, le champ magnétique peut être appliqué pendant une durée inférieure à celle provoquant l'orientation et/ou le déplacement définitif de la totalité des particules magnétiques de la région exposée.

Lorsque la clarté et/ou la couleur de la première composition changent progressivement sous l'effet du champ magnétique, l'utilisateur peut arrêter de soumettre les particules magnétiques au champ lorsque la première composition présente l'aspect souhaité.

Dans un exemple de mise en oeuvre de l'invention, le champ magnétique est exercé au travers d'une feuille magnétique. Selon la forme de celle-ci, les lignes de champ auront des géométries différentes, ce qui permet par exemple d'accroître le nombre de motifs susceptibles d'être réalisés avec un même aimant.

Le champ magnétique peut être exercé successivement sur différentes régions du support revêtues de la première composition.

Le champ magnétique peut être exercé sur des régions disjointes du support, afin par exemple de créer des motifs séparés.

Une région du support revêtue de la première composition peut être non exposée au champ magnétique, de manière à ne pas modifier dans cette région l'aspect de la première composition après son dépôt.

La première composition est appliquée au moyen d'un applicateur cosmétique non magnétique, choisi par exemple parmi les pinceaux, les embouts floqués et les mousses, la deuxième composition étant par exemple étalée avec les doigts ou pulvérisée.

Dans un exemple de mise en oeuvre de l'invention, l'une au moins des première et deuxième compositions est appliquée sur le support au travers d'un masque ajouré. Cela peut permettre par exemple de réaliser un motif prédéterminé, correspondant à la forme de l'ajour. Au moins une région du support recouverte par la première composition peut ensuite être exposée au champ magnétique.

### Exemples

L'invention pourra être mieux comprise à la lecture de la description détaillée qui va suivre, d'exemples non limitatifs de mise en oeuvre de celle-ci, ainsi qu'à l'examen du dessin annexé, sur lequel :
- la figure 1 représente, de manière schématique, un exemple de kit,
- la figure 2 représente en coupe axiale, schématique et partielle, le dispositif magnétique de la figure 1,
- les figures 3 et 4 illustrent de manière schématique l'utilisation du kit,
- la figure 5 représente un exemple de motif pouvant être obtenu grâce à l'invention,
- la figure 6 représente isolément, de manière schématique, un autre exemple de dispositif magnétique pouvant être utilisé,
- la figure 7 représente de manière schématique le dispositif magnétique de la figure 6 muni d'un organe de positionnement de l'aimant vis-à-vis du support maquillé,
- la figure 8 représente de manière schématique un autre exemple de kit,
- la figure 9 représente en élévation et isolément, de manière schématique, un exemple d'applicateur solidaire d'un dispositif magnétique,
- la figure 10 représente de manière schématique un autre exemple de dispositif magnétique pouvant être utilisé,
- la figure 11 représente un autre exemple de dispositif de conditionnement de la première composition,
- la figure 12 représente un masque ajouré pouvant être utilisé lors de la mise en oeuvre du procédé selon l'invention, et
- la figure 13 représente une feuille magnétique pouvant être utilisée lors de la mise en oeuvre du procédé selon l'invention.

Sur les figures, des particules magnétiques sont représentées sous la forme de points dans un souci de clarté du dessin, mais dans la réalité les particules peuvent ne pas être visibles à l'oeil nu.

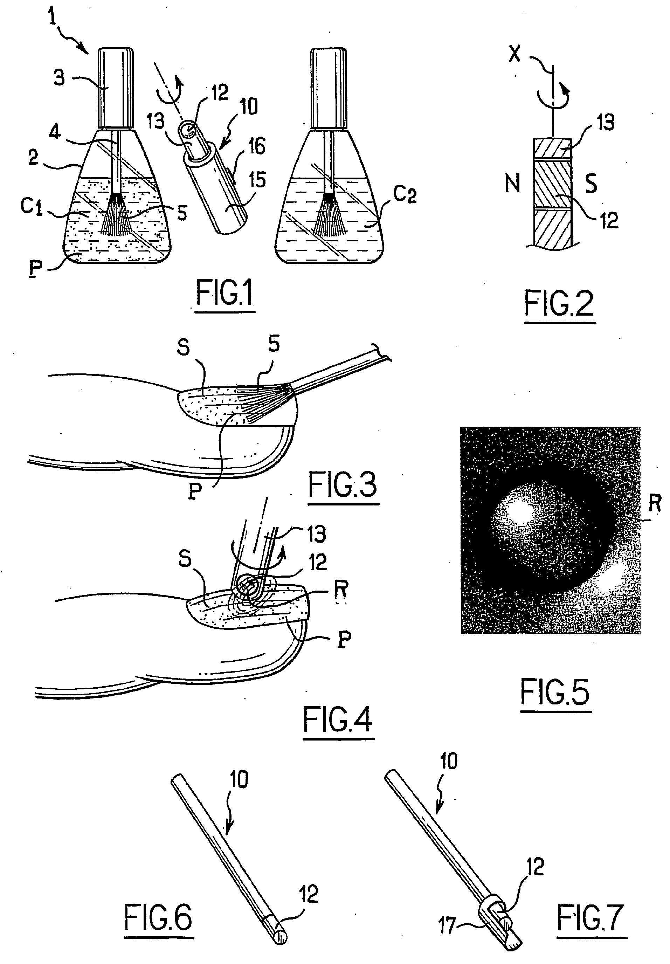

On a représenté sur la figure 1 un kit 1 comportant un première composition cosmétique C₁ comprenant des particules magnétiques P dont l'orientation et/ou la position conditionnent l'aspect de la composition après dépôt sur un support tel que la peau, les muqueuses ou les phanères ou encore des faux ongles.

Le kit 1 comporte également une deuxième composition C₂, contenue dans un récipient par exemple similaire à celui contenant la première composition C₁.

Dans l'exemple illustré, la composition C₁ est un vernis à ongles contenu dans un récipient 2 fermé par un capuchon 3. Ce dernier supporte un applicateur cosmétique 4, non magnétique, comportant un organe d'application 5 constitué par un pinceau permettant d'appliquer le vernis sur les ongles.

Le kit 1 comporte en outre un dispositif magnétique 10 permettant de générer un champ magnétique utile pour modifier l'aspect de la première composition C₁, sans contact avec celle-ci.

Dans l'exemple considéré, le dispositif magnétique 10 comporte un aimant permanent 12 supporté par un organe de maintien 13 d'axe longitudinal X, l'axe polaire de l'aimant 12 étant sensiblement perpendiculaire à l'axe X.

Dans l'exemple considéré, le dispositif magnétique 10 est agencé pour générer un champ magnétique tournant et comporte un moteur non apparent, logé dans un boîtier 15, pour entraîner en rotation l'organe de maintien 13 autour de son axe X.

Un interrupteur 16 est présent sur le boîtier 15 pour permettre à l'utilisateur de mettre en marche le moteur et d'entraîner en rotation l'organe de maintien 13 avec l'aimant 12.

Dans une variante de réalisation non illustrée, le champ magnétique tournant est généré par une pluralité de solénoïdes alimentés séquentiellement de manière à générer un champ tournant.

Pour utiliser le kit 1, l'utilisateur peut commencer, comme illustré sur la figure 3, par appliquer sur le support à maquiller S, en l'occurrence un ongle, la première composition C₁ à l'aide de l'applicateur 4.

Dans l'étape ultérieure illustrée sur la figure 4, l'utilisateur amène le dispositif magnétique 10 au-dessus d'une région centrale R du support S et actionne l'interrupteur 16 de manière à faire tourner l'aimant 12.

Les particules magnétiques contenues dans la première composition C₁ tendent à s'aligner avec les lignes de champ de l'aimant 12 et changent d'orientation, ce qui entraîne une modification de l'aspect de la composition C₁.

L'utilisateur peut choisir la durée d'application du champ magnétique en fonction du résultat qu'il souhaite obtenir.

Le motif obtenu peut par exemple donner l'impression d'une sphère en relief, comme illustré sur la figure 5.

L'utilisateur applique ensuite la deuxième composition C₂, qui est par exemple un vernis transparent, une fois que la première composition C₁ a séché.

L'application de cette deuxième composition C₂ peut permettre de créer un effet de profondeur supplémentaire, par exemple.

La première composition C₁ peut avoir dans l'exemple du vernis à ongles des figures 1 à 5 la formulation suivante, les quantités étant exprimées dans tous les exemples ci-après en fractions massiques.

### Exemple A

### Première composition

| | |
|---|---|
| Nitrocellulose | 11 |
| N-éthyl o,p-toluènesulfonamide | 5 |
| Résine alkyde | 10 |
| Isopropanol | 4 |
| Pigments magnétiques* | 0,5 |
| Acétate de butyle/acétate d'éthyle 50/50 | Qsp 100 |

| | |
|---|---|
| *Nacres contenant au moins 14 % de Fe₃O₄ de référence COLORONA PATINA GOLD (117288) commercialisées par la société MERCK. | |

L'aspect d'un tel vernis à ongles peut être modifié en appliquant un champ magnétique, avant que le vernis n'ait eu le temps de sécher.

Une deuxième composition C₂ ayant par exemple la formulation suivante peut être appliquée sur la première, après séchage de celle-ci.

### Deuxième composition

| | |
|---|---|
| Nitrocellulose | 11 |
| N-éthyl o,p-toluènesulfonamide | 5 |
| Résine alkyde | 10 |
| Isopropanol | 4 |
| Acétate de butyle/acétate d'éthyle 50/50 | Qsp 100 |

Dans une variante de mise en oeuvre de l'invention, la deuxième composition C₂ est appliquée avant la première composition C₁, par exemple pour créer un fond coloré.

La première composition C₁ peut alors être moins couvrante.

L'exemple suivant est un exemple de deuxième composition destinée à créer un fond coloré, la première composition ayant par exemple la formulation de l'exemple A ci-dessus.

### Exemple B

### Deuxième composition

| | |
|---|---|
| Nitrocellulose | 11 |
| N-éthyl o,p-toluènesulfonamide | 4 |
| Résine alkyde | 6 |
| Isopropanol | 4 |
| Pigments DC RED7 CI 15 850 | 2 |
| Acétate de butyle/acétate d'éthyle 50/50 | Qsp 100 |

La première composition de l'exemple A ne comporte qu'un seul type de pigments, magnétiques.

L'exemple suivant illustre la possibilité d'avoir au sein de la première composition des pigments magnétiques et une autre matière colorante, par exemple par des pigments à effet optique, en l'espèce des pigments goniochromatiques.

### Exemple C

### Première composition

| | |
|---|---|
| Nitrocellulose | 11 |
| N-éthyl o,p-toluènesulfonamide | 5 |
| Résine alkyde | 10 |
| Isopropanol | 4 |
| COLORONA BLACKSTAR GOLD, MERCE^{®} (pigments magnétiques) | 2,5 |
| SICOPEARL FANTASTICO ROSE, BASF^{®} (pigments goniochromatiques) | 2,5 |
| Acétate de butyle/acétate d'éthyle 50/50 | Qsp 100 |

Le pigment magnétique a la couleur or, laquelle est présente dans le trajet couleur du pigment goniochromatique.

Sans excitation magnétique, le mélange présente un effet goniochromatique sans motif apparent, la couleur du pigment magnétique permettant de ne pas éteindre l'effet goniochromatique. On peut observer une surface avec une couleur de fond variant de l'or au rose.

En revanche, après application d'un champ magnétique, les pigments magnétiques s'alignant sur les lignes de champ créent un motif qui vient se superposer au changement de couleur apporté par le pigment goniochromatique. Un motif de couleur or, obtenu grâce aux particules de pigment magnétique orientées, peut apparaître sur un fond rose pour certaines conditions d'orientation de l'observation et/ou du support maquillé.

Une deuxième composition ayant par exemple la même formulation que celle de l'exemple A peut être appliquée sur la première, après séchage de celle-ci.

La première composition peut encore, par exemple, contenir des pigments magnétiques et des pigments diffractants.

### Exemple D

### Première composition

| | |
|---|---|
| Nitrocellulose | 11 |
| N-éthyl o,p-toluènesulfonamide | 5 |
| Résine alkyde | 10 |
| Isopropanol | 4 |
| Pigments magnétiques* | 0,5 |
| Pigments à effet optique diffractant** | 3 |
| Acétate de butyle/acétate d'éthyle 50/50 | Qsp 100 |

| | |
|---|---|
| *Nacres contenant au moins 14 % de Fe₃O₄ de référence COLORONA PATINA GOLD (117288) commercialisées par la société MERCK. **Pigments commercialisés sous la dénomination SPECTRAFLAIR par la société FLEXPRODUCTS. | |

La deuxième composition est par exemple celle de l'exemple A.

Bien entendu, quelle que soit la nature des première et deuxième compositions, le champ magnétique appliqué à la première composition peut être autre que tournant. A titre d'exemple, on a représenté à la figure 6 un dispositif magnétique qui comporte un aimant permanent 12 en forme de barreau à son extrémité.

Lorsque le champ magnétique n'est pas tournant, l'utilisateur peut par exemple déplacer l'aimant à proximité de la première composition en fonction du résultat qu'il souhaite obtenir.

Le dispositif magnétique, quel qu'il soit, peut comporter un organe permettant de le positionner relativement au support S.

Cet organe de positionnement sert par exemple à éviter que le dispositif magnétique ne touche la composition pendant que le champ magnétique est exercé.

L'organe de positionnement peut servir encore à centrer le motif qui est réalisé relativement au support S, par exemple l'ongle.

Selon la nature du support, l'organe de positionnement pourra prendre diverses formes, par exemple celle d'une extension 17 offrant une surface de butée pour l'extrémité du doigt, comme illustré sur la figure 7.

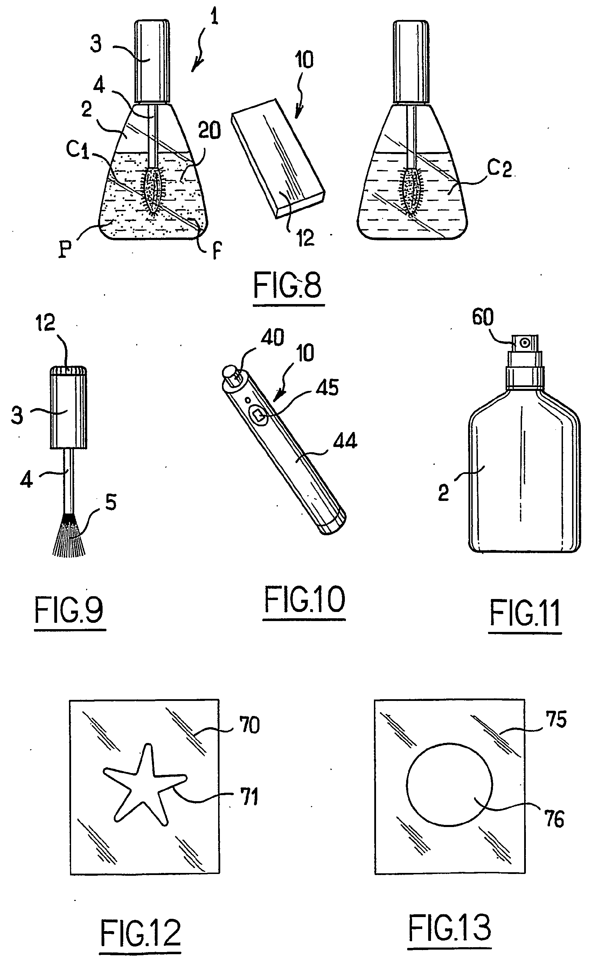

On a représenté sur la figure 8 un autre exemple de kit 1 selon l'invention, comportant une première composition C₁ et une deuxième composition C₂, respectivement constituées dans cet exemple par un rouge à lèvres liquide et un brillant à lèvres.

L'applicateur 4 comporte dans cet exemple un embout floqué 20 supporté par le capuchon 3 du récipient 2.

Le dispositif magnétique 10 se présente par exemple sous la forme d'une structure souple, par exemple en matière plastique chargée de particules aimantées, créant une alternance de pôles N et S, ce qui permet de former sur le support revêtu de la première composition des motifs répétés, par exemple des rayures.

Dans le cas d'un rouge à lèvres, la première composition C₁ présente par exemple la formulation suivante.

### Exemple E

### Première composition

| | |
|---|---|
| Octyl-2 dodécanol | 10 |
| Ditertiobutyl 4-hydroxytoluene | 0,07 |
| Polybutène (monooléfines / isoparaffines 95/5) (PM : 2060) | 50 |
| Mélange de p-hydroxybenzoates d'isopropyle, iso-butyle, n-butyle (40/30/30) | 0,4 |
| Tetra-iso-stéarate de pentaérythrityle | 11,33 |
| Tri-mellitate de tridécyle | 13 |
| Triglycéride d'acide 2-décyl tétradécanoïque (GUERBET C24) | 15 |
| Pigments magnétiques* | 0,2 |

| | |
|---|---|
| * Nacres contenant au moins 14 % de Fe₃O₄ commercialisées sous la référence CLOISONNE NU ANTIQUE GREEN 828 CB par la société ENGELHARD. | |

La deuxième composition présente par exemple la formulation suivante.

### Deuxième composition

| | |
|---|---|
| Aérosil R972 de Degussa | 5 |
| Polyisobutylène hydrogéné (huile de Parleam) | 2.1 |
| Octyldodécanol | 0.9 |
| Huile de silicone phénylée (Dow Coming 556C) | 2.1 |
| Copolymère de polyvinyl pyrrolidone/Eicosène (Anatron V220 d'ISP) | 1.2 |
| Isododécane | Qsp 100 |

La deuxième composition peut être appliquée sur la première et permet de créer un effet de profondeur.

Quel que soit le type d'applicateur, l'aimant 12 peut, le cas échéant, être incorporé à l'applicateur.

Dans l'exemple de la figure 9, le capuchon 3 de fermeture est surmonté par l'aimant 12, du côté opposé à l'organe d'application 5.

Dans l'exemple de la figure 13, l'aimant 12 est supporté par un organe de maintien 13 surmonté d'un capuchon 51 et peut se loger en l'absence d'utilisation dans un compartiment 50 du capuchon 3 permettant de fermer le récipient 2 contenant la première composition C₁. Le capuchon 51 sert à la préhension de l'aimant 12 et permet de fermer le compartiment 50.

On ne sort pas du cadre de la présente invention lorsque le champ magnétique est généré par un électroaimant au lieu d'un aimant permanent.

Sur la figure 10, on a représenté un dispositif magnétique 10 comportant un électroaimant 40 à une extrémité d'un boîtier 44 logeant l'alimentation électrique.

Un interrupteur 45 permet à l'utilisateur d'alimenter sélectivement l'électroaimant 40.

Divers dispositifs autres que ceux qui viennent d'être décrits permettant de conditionner et/ou de distribuer ou appliquer les compositions C₁ et C₂ peuvent être utilisés.

L'une au moins des compositions C₁ et C₂ peut par exemple être déposée sur le support S sans applicateur mais sous forme de spray, par exemple en utilisant une pompe 60 comme illustré à la figure 11. Le spray peut encore être généré au moyen d'un aérographe ou d'un récipient pressurisé, par exemple.

Les dispositifs de conditionnement et/ou de distribution ou application des première et deuxième compositions peuvent différer l'un de l'autre.

Un masque ajouré 70, représenté sur la figure 12 et comportant comme ajour un motif 71, sous forme d'étoile dans l'exemple illustré, peut être interposé entre le spray et le support à maquiller.

Une feuille 75 perméable au champ magnétique, ajourée ou non, peut être interposée entre l'aimant 12 ou l'électroaimant 40 et le support S, de manière à modifier la géométrie des lignes de champ et créer de nouveaux effets, comme illustré sur la figure 13.

Bien entendu, l'invention n'est pas limitée aux exemples qui viennent d'être décrits.

Par exemple, le kit peut comporter une pluralité d'aimants ayant des formes différentes de manière à réaliser des motifs différents.

Dans toute la description, y compris les revendications, l'expression "comportant un" doit être comprise comme étant synonyme de "comportant au moins un", sauf si le contraire est spécifié.

## Revendications

1. Procédé de maquillage d'un support (S), tel que la peau, les phanères ou les muqueuses, comportant les étapes suivantes :
- déposer sur le support (S) au moins des première et deuxième compositions cosmétiques à l'état fluide, la première composition recouvrant ou étant recouverte au moins partiellement par la deuxième, la première composition (C₁) contenant des particules magnétiques (P) mobiles sous l'effet d'un champ magnétique, la première composition (C₁) étant appliquée au moyen d'un applicateur (4) cosmétique l'applicateur (4) étant non magnétique,
- exposer au moins partiellement la première composition (C₁) à un champ magnétique, de manière à modifier l'orientation et/ou déplacer au moins une partie des particules magnétiques (P).

2. Procédé selon la revendication 1, **caractérisé par le fait que** le champ magnétique est appliqué de manière à former au moins un motif sur la première composition (C₁).

3. Procédé selon la revendication 1, **caractérisé par le fait que** le champ magnétique est appliqué de manière à modeler la clarté et/ou la couleur d'une région au moins du visage ou du corps sur laquelle la première composition (C₁) a été appliquée.

4. Procédé selon l'une quelconque des revendications 1 à 3, **caractérisé par le fait que** le champ magnétique est exercé par un aimant (12) permanent.

5. Procédé selon la revendication précédente, **caractérisé par le fait que** l'aimant (12) est entraîné en rotation.

6. Procédé selon l'une quelconque des revendications 1 à 3, **caractérisé par le fait que** le champ magnétique est exercé par un électroaimant (40).

7. Procédé selon la revendication 6, **caractérisé par le fait que** l'électroaimant (40) est alimenté par au moins une pile ou un accumulateur.

8. Procédé selon l'une des revendications 6 et 7, **caractérisé par le fait que** l'électroaimant (40) est alimenté sélectivement par l'utilisateur.

9. Procédé selon l'une quelconque des revendications 1 à 8, **caractérisé par le fait que** le champ magnétique est appliqué jusqu'à obtenir un aspect figé de la première composition (C₁).

10. Procédé selon l'une quelconque des revendications 1 à 8, **caractérisé par le fait que** le champ magnétique est appliqué pendant une durée inférieure à celle provoquant l'orientation et/ou le déplacement définitif de la totalité des particules magnétiques de la région (R) exposée.

11. Procédé selon l'une quelconque des revendications 1 à 10, **caractérisé par le fait que** le champ magnétique est exercé successivement sur différentes régions (R) du support (S) revêtues de la première composition (C₁).

12. Procédé selon la revendication précédente, **caractérisé par le fait que** le champ magnétique est exercé sur des régions disjointes du support (S).

13. Procédé selon l'une quelconque des revendications 1 à 12, **caractérisé par le fait qu'**une région au moins du support (S) revêtue de la première composition est non exposée au champ magnétique.

14. Procédé selon la revendication 1, **caractérisé par le fait que** l'applicateur (4) comporte un pinceau (5), un embout floqué (20) ou une mousse.

15. Procédé selon l'une quelconque des revendications 1 à 14, **caractérisé par le fait que** les particules magnétiques (P) comportent un matériau magnétique choisi dans le groupe constitué par : le fer, le nickel, le cobalt, leurs alliages et oxydes.

16. Procédé selon l'une quelconque des revendications 1 à 15, **caractérisé par le fait que** la première composition (C₁) comporte des particules magnétiques et des particules non magnétiques.

17. Procédé selon l'une quelconque des revendications 1 à 16, **caractérisé par le fait que** la première composition (C₁) est appliquée sur le support (S) au travers d'un masque (70) ajouré.

18. Procédé selon l'une quelconque des revendications 1 à 17, **caractérisé par le fait que** le champ magnétique est appliqué au travers d'une feuille (75) perméable au champ magnétique.

19. Procédé selon l'une quelconque des revendications 1 à 18, **caractérisé par le fait que** la première composition (C₁) prend un état empêchant tout changement d'orientation des particules magnétiques (P) sous l'effet d'un champ magnétique après une durée de séchage donnée.

20. Procédé selon l'une quelconque des revendications 1 à 19, **caractérisé par le fait que** la deuxième composition (C₂) est appliquée sur la première.

21. Procédé selon l'une quelconque des revendications 1 à 19, **caractérisé par le fait que** la première composition (C₁) est appliquée sur la deuxième.

22. Procédé selon l'une quelconque des revendications 1 à 21, **caractérisé par le fait que** la deuxième composition est incolore.

23. Procédé selon l'une quelconque des revendications 1 à 21, **caractérisé par le fait que** la deuxième composition est colorée.

24. procédé selon l'une quelconque des revendications 1 à 22, **caractérisé par le fait que** la deuxième composition est transparente.

## Claims

1. A method of applying makeup to a surface (S), such as the skin, the nails, hair, or the lips, said method comprising the following steps:
· depositing at least first and second cosmetic compositions in the fluid state on the surface (S), the first composition covering or being covered at least in part by the second, the first composition (C₁) containing magnetic particles (P) that are movable under the effect of a magnetic field; the first composition (C₁) being applied by means of a cosmetics applicator (4), the applicator (4) being non-magnetic,
· exposing at least part of the first composition (C₁) to a magnetic field, so as to change the orientation of and/or displace at least a fraction of the magnetic particles (P).

2. A method according to claim 1, **characterized by** the fact that the magnetic field is applied so as to form at least one pattern on the first composition (C₁).

3. A method according to claim 1, **characterized by** the fact that the magnetic field is applied so as to model the clarity and/or the color of at least a region of the face or of the body on which the first composition (C₁) has been applied.

4. A method according to any one of claims 1 to 3, **characterized by** the fact that the magnetic field is exerted by a permanent magnet (12).

5. A method according to the preceding claim, **characterized by** the fact that the magnet (12) is rotated.

6. A method according to any one of claims 1 to 3, **characterized by** the fact that the magnetic field is exerted by an electromagnet (40).

7. A method according to claim 6, **characterized by** the fact that the electromagnet (40) is powered by at least one optionally-rechargeable battery.

8. A method according to claim 6 or claim 7, **characterized by** the fact that the electromagnet (40) is switched on selectively by the user.

9. A method according to any one of claims 1 to 8, **characterized by** the fact that the magnetic field is applied until the first composition (C₁) obtains a fixed appearance.

10. A method according to any one of claims 1 to 8, **characterized by** the fact that the magnetic field is applied for a period of time that is shorter than the period of time that causes all of the magnetic particles in the exposed region (R) to be permanently displaced and/or oriented.

11. A method according to any one of claims 1 to 10, **characterized by** the fact that the magnetic field is exerted successively on different regions (R) of the surface (S) that are coated with the first composition (C₁).

12. A method according to the preceding claim, **characterized by** the fact that the magnetic field is exerted on regions of the surface (S) that are disjoint.

13. A method according to any one of claims 1 to 12, **characterized by** the fact that at least one region of the surface (S) that is coated with the first composition is not exposed to the magnetic field.

14. A method according to claim 1, **characterized by** the fact that the applicator (4) comprises a brush (5), a flocked endpiece (20), or a foam.

15. A kit according to any one of claims 1 to 14, **characterized by** the fact that the magnetic particles (P) comprise a magnetic material selected from the group constituted by: iron, nickel, cobalt, and alloys and oxides thereof.

16. A method according to any one of claims 1 to 15, **characterized by** the fact that the first composition (C₁) contains magnetic particles and non-magnetic particles.

17. A method according to any one of claims 1 to 16, **characterized by** the fact that the first composition (C₁) is applied to the surface (S) through a perforated mask (70).

18. A method according to any one of claims 1 to 17, **characterized by** the fact that the magnetic field is applied through a sheet (75) that is permeable to the magnetic field.

19. A method according to any one of claims 1 to 18, **characterized by** the fact that, after a given drying time, the first composition (C₁) takes on a state that prevents the magnetic particles (P) from changing their orientation under the effect of a magnetic field.

20. A method according to any one of claims 1 to 19, **characterized by** the fact that the second composition (C₁) is applied to the first.

21. A method according to any one of claims 1 to 19, **characterized by** the fact that the first composition (C₁) is applied to the second.

22. A method according to any one of claims 1 to 21, **characterized by** the fact that the second composition is colorless.

23. A method according to any one of claims 1 to 21, **characterized by** the fact that the second composition is colored.

24. A method according to any one of claims 1 to 22, **characterized by** the fact that the second composition is transparent.

## Patentansprüche

1. Verfahren zum Schminken eines Trägers (S), wie der Haut, der Hautanhangsorgane oder der Schleimhäute, umfassend die folgenden Schritte:
- auf den Träger (S) mindestens eine erste und eine zweite kosmetische Zusammensetzung in fließfähigem Zustand aufbringen, wobei die erste Zusammensetzung mindestens teilweise die zweite bedeckt oder von dieser bedeckt wird, wobei die erste Zusammensetzung (C₁) magnetische Teilchen (P) enthält, die unter der Einwirkung eines Magnetfelds beweglich sind, wobei die erste Zusammensetzung (C₁) mit Hilfe eines kosmetischen Applikators (4) aufgetragen wird, wobei der Applikator (4) nicht magnetisch ist.
- die erste Zusammensetzung (C₁) mindestens teilweise einem Magnetfeld ausgesetzt wird, so dass die Ausrichtung geändert wird und/oder mindestens ein Teil der magnetischen Teilchen (P) verlagert wird.

2. Verfahren nach Anspruch 1, **dadurch gekennzeichnet, dass** das Magnetfeld so angelegt wird, dass auf der ersten Zusammensetzung (C₁) mindestens ein Muster gebildet wird.

3. Verfahren nach Anspruch 1, **dadurch gekennzeichnet, dass** das Magnetfeld so angelegt wird, dass die Helligkeit und/oder die Farbe mindestens eines Bereichs des Gesichts oder des Körpers, auf den die erste Zusammensetzung (C₁) aufgetragen wurde, modelliert wird.

4. Verfahren nach einem der Ansprüche 1 bis 3, **dadurch gekennzeichnet, dass** das Magnetfeld durch einen Dauermagnet (12) ausgeübt wird.

5. Verfahren nach dem vorhergehenden Anspruch, **dadurch gekennzeichnet, dass** der Magnet (12) in Drehung versetzt wird.

6. Verfahren nach einem der Ansprüche 1 bis 3, **dadurch gekennzeichnet, dass** das Magnetfeld durch einen Elektromagnet (40) ausgeübt wird.

7. Verfahren nach Anspruch 6, **dadurch gekennzeichnet, dass** der Elektromagnet (40) durch mindestens eine Batterie oder einen Akku versorgt wird.

8. Verfahren nach einem der Ansprüche 6 und 7, **dadurch gekennzeichnet, dass** der Elektromagnet (40) durch den Benutzer wahlweise versorgt wird.

9. Verfahren nach einem der Ansprüche 1 bis 8, **dadurch gekennzeichnet, dass** das Magnetfeld angelegt wird, bis man ein unveränderliches Aussehen der ersten Zusammensetzung (C₁) erhält.

10. Verfahren nach einem der Ansprüche 1 bis 8, **dadurch gekennzeichnet, dass** das Magnetfeld während einer Zeit angelegt wird, die kleiner als diejenige ist, die die Ausrichtung und/oder die endgültige Verlagerung der Gesamtheit der magnetischen Teilchen des ausgesetzten Bereichs (R) bewirkt.

11. Verfahren nach einem der Ansprüche 1 bis 10, **dadurch gekennzeichnet, dass** das Magnetfeld nacheinander auf verschiedene mit der ersten Zusammensetzung (C₁) bedeckte Bereiche (R) des Trägers (S) ausgeübt wird.

12. Verfahren nach dem vorhergehenden Anspruch, **dadurch gekennzeichnet, dass** das Magnetfeld auf getrennte Bereiche des Trägers (S) ausgeübt wird.

13. Verfahren nach einem der Ansprüche 1 bis 12, **dadurch gekennzeichnet, dass** mindestens ein mit der ersten Zusammensetzung bedeckter Bereich des Trägers (S) nicht dem Magnetfeld ausgesetzt wird.

14. Verfahren nach Anspruch 1, **dadurch gekennzeichnet, dass** der Applikator (4) einen Pinsel (5), einen beflockten Ansatz (20) oder einen Schaum umfasst.

15. Verfahren nach einem der Ansprüche 1 bis 14, **dadurch gekennzeichnet**, die die magnetischen Teilchen (P) einen magnetischen Werkstoff umfassen, der aus der Gruppe ausgewählt ist, bestehend aus: Eisen, Nickel, Kobalt, ihre Legierungen und Oxide.

16. Verfahren nach einem der Ansprüche 1 bis 15, **dadurch gekennzeichnet, dass** die erste Zusammensetzung (C₁) magnetische Teilchen und nicht-magnetische Teilchen umfasst.

17. Verfahren nach einem der Ansprüche 1 bis 16, **dadurch gekennzeichnet, dass** die erste Zusammensetzung (C₁) auf den Träger (S) durch eine durchbrochene Maske (70) hindurch aufgetragen wird.

18. Verfahren nach einem der Ansprüche 1 bis 17, **dadurch gekennzeichnet, dass** das Magnetfeld durch ein für das Magnetfeld durchlässiges Blatt (75) hindurch angelegt wird.

19. Verfahren nach einem der Ansprüche 1 bis 18, **dadurch gekennzeichnet, dass** die erste Zusammensetzung (C₁) einen Zustand annimmt, der nach einer gegebenen Trocknungszeit jede Ausrichtungsänderung der magnetischen Teilchen (P) unter der Einwirkung des Magnetfelds verhindert.

20. Verfahren nach einem der Ansprüche 1 bis 19, **dadurch gekennzeichnet, dass** die zweite Zusammensetzung (C₂) auf die erste aufgetragen wird.

21. Verfahren nach einem der Ansprüche 1 bis 19, **dadurch gekennzeichnet, dass** die erste Zusammensetzung (C₁) auf die zweite aufgetragen wird.

22. Verfahren nach einem der Ansprüche 1 bis 21, **dadurch gekennzeichnet, dass** die zweite Zusammensetzung farblos ist.

23. Verfahren nach einem der Ansprüche 1 bis 21, **dadurch gekennzeichnet, dass** die zweite Zusammensetzung gefärbt ist.

24. Verfahren nach einem der Ansprüche 1 bis 22, **dadurch gekennzeichnet, dass** die zweite Zusammensetzung transparent ist.
